# EUROPEAN PATENT APPLICATION

(11) **EP 2 018 886 A1**
(43) Date of publication of application: **28.01.2009**
(21) Application number: 07816792.1
(22) Date of filing: 09.11.2007
(51) Int. Cl.: A61M 15/00, A61M 11/00

(54) **AEROSOLIZING INHALATION DEVICE**

(30) Priority: 10.11.2006 CN 200620094038 U
(71) Applicant: Han, Li, Hong Kong (CN)
(72) Inventor: Han, Li, Hong Kong (CN)
(74) Representative: Wachinger, Julian Friedrich
(86) International application number: PCT/CN2007/003180
(87) International publication number: WO 2008/055423

(57) **Abstract**

An aerosolizing inhalation device for aerosolizing the solution with the substance of physiologic activity into aerosol and delivering it into lungs via the respiratory tract, includes a housing (2), and a battery (3), a pneumatic switch (4), an atomizer (6) and a liquid-supplying bottle (7), all of which are arranged in turn and disposed in the housing (2). The liquid-supplying bottle (7) is provided with a vent duct (8), and one end of the liquid-supplying bottle (7) is connected to the atomizer (6). The housing (2) is provided with an air orifice (5). The aerosolizing inhalation device can also be used as a cigarette substitute.

## Description

### Technical Field

This utility model relates to an inhalation device, in particular, an aerosolizing inhalation device that aerosolizes the solution with the substance of physiologic activity into aerosol and delivers it into lungs via the respiratory tract.

### Background Art

For the existing aerosolizing lung inhalation devices for medical purposes, a broad range of ultrasonic atomizers are available for clinic and family use. However, the diameter of aerosol particles from the existing aerosolizing devices is too big, and most aerosol particles are retained by the upper respiratory tract and bronchus. As a result, they can't effectively reach the bronchiole or alveolus. In addition, such devices are not easy to carry due to their big sizes.

### Contents of utility model

To overcome the disadvantages associated with the above-mentioned prior art, this utility model provides an aerosolizing inhalation device, which, featuring small aerosol particles, even aerosolizing effect, small size and portability, are suitable for outdoor medical care and rescue.

The major technical solution of this utility model is as follows: an aerosolizing inhalation device includes a housing, and a battery, a pneumatic switch, an atomizer and a liquid-supplying bottle, all of which are arranged in turn and disposed in the housing. The liquid-supplying bottle is provided with a vent duct, and one end of the liquid-supplying bottle contacts with the atomizer. The housing is provided with an air orifice. ,

This utility model also adopts the following auxiliary technical solution: the said air orifice is located between the said pneumatic switch and atomizer.

The said atomizer is a jet heating atomizer, which includes a liquid-supplying structure and heating body. The said liquid-supplying structure is a porous structure or fiber structure, on one end of which there is a jet hole, while on the other end, there is a protuberance. The said liquid-supplying bottle contacts the said protuberance, so that the aerosolizing liquid can penetrate into the said liquid-supplying structure. The said heating body is located inside the liquid-supplying structure and electrically connected with the battery.

The said atomizer is a impregnation heating atomizer, which includes a liquid-supplying structure and heating body. The said liquid-supplying structure is a porous structure or fiber structure, one end of which forms a protuberance, while on the other end, the said heating body is installed. The said heating body is electrically connected with the battery. The said liquid-supplying bottle contacts the protuberance, so that the aerosolizing liquid can penetrate into the said liquid-supplying structure.

The said liquid-supplying structure is a porous structure or fiber structure. The heating body may be a micro-porous ceramic framework with Ohmic metal electro-thermal material or a porous component created with electrically conductive ceramics / PTC material, which is associated with sintered electrode.

The said pneumatic switch is a mechanical switch made of rubber film and metal and driven by pressure difference. Alternatively, it may be a Hall element, semiconductor force-sensitive chip, silicon matrix bridge chip, capacitance or induction sensor.

The said liquid-supplying bottle is a porous structure or fiber structure, and is filled with aerosolizing liquid.

There is an indicator on the front end of the said housing.

This utility model will bring the following helpful effects:
1. For this utility model, the atomizer is a jet heating atomizer, which carries out the primary atomization in the jet hole (103) with the suction airflow, and then the secondary atomization by spraying onto the heating body (102). The atomization for twice enables even atomization, and small aerosolized particles with a mean diameter of about 1µm, which is the most optimal technical parameter for lung inhalation, allowing for total inhalation.
2. For this utility model, the atomizer has its battery, pneumatic switch, atomizer, and liquid-supplying bottle arranged in the housing in turn, featuring compact structure, small size, portability, and no restriction on application site. It is suitable for outdoor medical care and rescue.

### Description of Drawings

Figure 1 is the structure diagram of this utility model.
Figure 2 is the structure diagram of the jet heating atomizer in the first example of this utility model.
Figure 3 is the right view of Figure 2.
Figure 4 is the structure diagram of the impregnation atomizer in the second example of this utility model.
Figure 5 is the right view of Figure 4.

### Specific Mode for Carrying Out the Utility model

This utility model is further described as follows on the basis of the drawings.

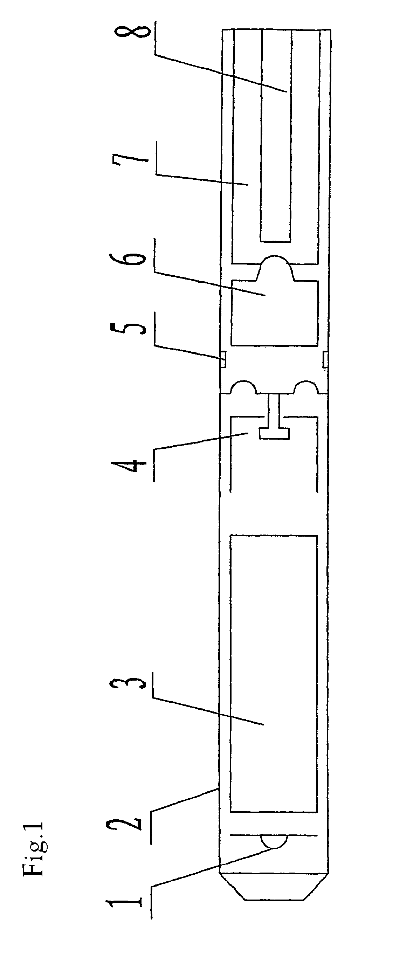

As shown in Figure 1, this utility model includes a housing (2), and a battery (3), a pneumatic switch (4), an atomizer (6) and a liquid-supplying bottle (7), all of which are arranged in turn and disposed in the housing (2), which is a hollow cylinder, on the front of which is an indicator (1). The liquid-supplying bottle (7) is provided with a vent duct (8), and one end of the liquid-supplying bottle (7) is connected to the atomizer (6). On the other side of the atomizer (6), there are the pneumatic switch (4) and battery (3) successively. The battery (3), pneumatic switch (4) and atomizer (6) are in series connection to form a loop. The pneumatic switch (4) controls the operation of the atomizer (6). In this example, the pneumatic switch (4) is a mechanical switch made of rubber film and metal and driven by pressure difference. Alternatively, it may be a Hall element, semiconductor force-sensitive chip, silicon matrix bridge chip, capacitance or induction sensor. The housing (2) is provided with an air orifice (5) between the pneumatic switch (4) and atomizer (6) for air inhalation from outside.

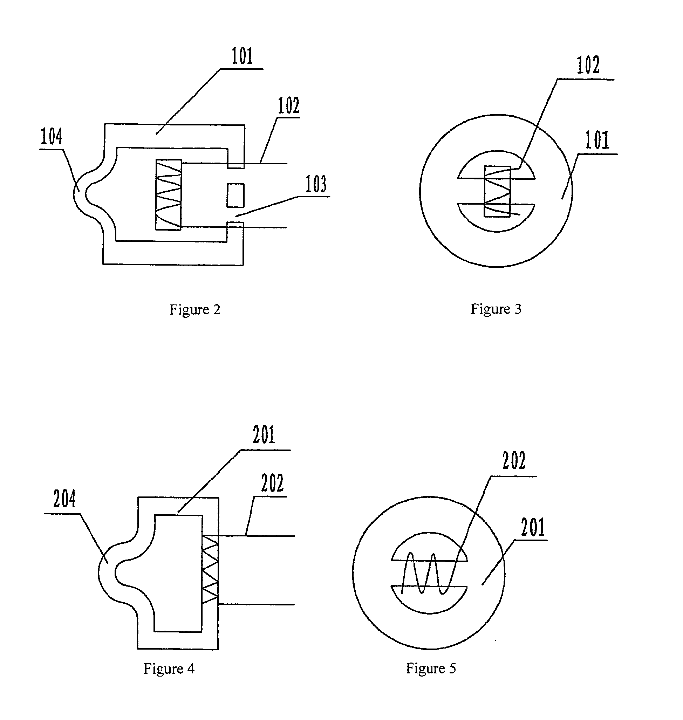

As shown in Figure 2 and 3, the first example of this utility model, in which the atomizer (6) is a jet heating atomizer that includes the liquid-supplying structure (101) and heating body (102). The liquid-supplying structure (101) is a porous structure or fiber structure, on one end of which there is a jet hole (103), while on the other end, there is a protuberance (104). The jet hole (103) is made of foamed ceramics, micro-porous ceramics, foamed metal, stainless steel fiber felt or chemical fiber through molding and drilling. The heating body (102) is located inside the liquid-supplying structure (101), electrically connected with the battery (3), and controlled with the pneumatic switch (4). The liquid-supplying bottle (7) contacts the protuberance (104), so that the aerosolizing liquid can penetrate into the liquid-supplying structure (101). The heating body (102) is a micro-porous ceramic framework with Ohmic metal electro-thermal material or a porous component created with electrically conductive ceramics / PTC material, which is associated with sintered electrode. The jet heating atomizer carries out the primary atomization in the jet hole (103) with the suction airflow, and then the secondary atomization by spraying onto the heating body (102). The atomization for twice enables even atomization, and small aerosolized particles with a mean diameter of about 1µm, which is the most optimal technical parameter for lung inhalation.

As shown in Figure 4 and 5 , the second example of this utility model, in which the atomizer (6) is an impregnation atomizer, which includes the liquid-supplying structure (201) and heating body (202). The liquid-supplying structure (201) is a porous structure or fiber structure, on one end of which is a protuberance (204), while on the other end is the heating body (202). The heating body (202) is electrically connected with the battery (3), and controlled with the pneumatic switch (4). The liquid-supplying bottle (7) contacts the protuberance (204), so that the aerosolizing liquid can penetrate into the liquid-supplying structure (201). The heating body (202) is a micro-porous ceramic framework with Ohmic metal electro-thermal material or a porous component created with electrically conductive ceramics / PTC (Positive Temperature Coefficient) material, which is associated with sintered electrode. The impregnation atomizer leverages the heating body (202) to directly heat the liquid-supplying structure (201), thus aerosolizing the solution.

On one end of the atomizer (6) in the above-mentioned two examples, the protuberances (104, 204) are connected with the liquid-supplying bottle (7). The aerosolizing solution in the liquid-supplying bottle (7) enters the atomizer (6) through the liquid-supplying structure (101, 201). The liquid-supplying structure (101, 201) is made of foamed ceramics, micro-porous ceramics, perforated metal, stainless steel fiber felt or chemical fiber through molding and drilling. The heating body (102, 202) is a micro-porous ceramic framework with Ohmic metal electro-thermal material or a porous component directly created with electrically conductive ceramics / PTC material, which is associated with sintered electrode. The surface of the heating body (101, 202) is sintered into high-temperature glaze to fix the zeolite grains, which are made of natural zeolite, artificial non-organic micro-porous ceramics or aluminum oxide grains.

The operating principle of this utility model is as follows: The medicine is dissolved with propanediol, and the aerosolizing solution is put into the changeable liquid-supplying bottle (7). The liquid-supplying bottle (7) is put into the housing (10), contacts the atomizer (6), and supplies the liquid to it. When the user sucks, the pneumatic switch (4) accomplishes the mechanical contact under the action of pressure difference, connecting the battery (3) with the atomizer (6). The battery (3) electrifies the atomizer (6), and the heating body (102, 202) starts to heat. When the user sucks, the indicator (1) is lit, and the atomizer (6) condenses and dilutes the air in the liquid-supplying bottle (7), thus making mist, which is sucked out through the vent duct (8) on the liquid-supplying bottle.

The fiber structure for production of the liquid-supplying bottle and liquid-supplying structure is made of chemical fiber or nylon fiber.

The atomizer of this utility model atomizes the aerosolizing solution impregnated from the liquid-supplying bottle, and the mist is diluted with the inhaled air into aerosol, which is inhaled by the user. This utility model may be used as cigarette substitute and medical device delivering medicine to lungs.

## Claims

1. An aerosolizing inhalation device includes a housing (2), and a battery (3), a pneumatic switch (4), an atomizer (6) and a liquid-supplying bottle (7), all of which are arranged in turn and disposed in the housing (2); the liquid-supplying bottle (7) is provided with a vent duct (8), and one end of the liquid-supplying bottle (7) is connected to the atomizer (6); the housing (2) is provided with an air orifice (5).

2. The aerosolizing inhalation device of Claim 1, wherein the said air orifice (5) is located between the said pneumatic switch (4) and atomizer (6).

3. The aerosolizing inhalation device of Claim 1 or 2, wherein the said atomizer (6) is a jet heating atomizer, which includes a liquid-supplying structure (101) and heating body (102);the said liquid-supplying structure (101) is a porous structure or fiber structure, on one end of which there is a jet hole (103), while on the other end, there is a protuberance (104);the said liquid-supplying bottle (7) contacts the said protuberance (104), so that the aerosolizing liquid can penetrate into the said liquid-supplying structure (101);the said heating body (102) is located inside the liquid-supplying structure (101) and electrically connected with the battery (3).

4. The aerosolizing inhalation device of Claim 1 or 2, wherein the said atomizer (6) is an impregnation atomizer, which includes the liquid-supplying structure (201) and heating body (202); the liquid-supplying structure (201) is a porous structure or fiber structure, on one end of which is a protuberance (204), while on the other end is the heating body (202);the said heating body (202) is electrically connected with the battery (3), and the said liquid-supplying bottle (7) contacts the protuberance (204), so that the aerosolizing liquid can penetrate into the liquid-supplying structure (201).

5. The aerosolizing inhalation device of Claim 3, wherein the said liquid-supplying structure (101, 201) is a porous structure or fiber structure, and that the heating body (102, 202) is a micro-porous ceramic framework with Ohmic metal electro-thermal material or a porous component created with electrically conductive ceramics / PTC material, which is associated with sintered electrode.

6. The aerosolizing inhalation device of Claim 4 , wherein the said liquid-supplying structure (101, 201) is a porous structure or fiber structure, and that the heating body (102, 202) is a micro-porous ceramic framework with Ohmic metal electro-thermal material or a porous component created with electrically conductive ceramics / PTC material, which is associated with sintered electrode.

7. The aerosolizing inhalation device of Claim 1 or 2, wherein the said pneumatic switch (4) is a mechanical switch made of rubber film and metal and driven by pressure difference;alternatively, it may be a Hall element, semiconductor force-sensitive chip, silicon matrix bridge chip, capacitance or induction sensor.

8. The aerosolizing inhalation device of Claim 1 or 2, wherein the said liquid-supplying structure (7) is a porous structure or fiber structure, which is filled with aerosolizing solution.

9. The aerosolizing inhalation device of Claim 5, wherein the said liquid-supplying structure (7) is a porous structure or fiber structure, which is filled with aerosolizing solution.

10. The aerosolizing inhalation device of Claim 6, wherein the liquid-supplying structure (7) is a porous structure or fiber structure, which is filled with aerosolizing solution.

11. The aerosolizing inhalation device of Claim 10, wherein the said housing (2) has an indicator (1) on its front end.
